# EUROPÄISCHE PATENTSCHRIFT

(11) **EP 3 496 685 B1**
(45) Veröffentlichungstag und Bekanntmachung des Hinweises auf die Patenterteilung: **28.09.2022**
(21) Anmeldenummer: 17749707.0
(22) Anmeldetag: 09.08.2017
(51) Int. Cl.: A61B 1/018, A61B 17/00, A61F 13/00, A61M 1/00

(54) **FASSSCHLAUFE FÜR SAUGKÖRPER ZUR ENDOLUMINALEN UNTERDRUCKTHERAPIE**
HOLDING LOOP FOR SUCTION BODIES FOR ENDOLUMINAL NEGATIVE PRESSURE THERAPY
BOUCLE DE PRÉHENSION POUR CORPS D'ASPIRATION SERVANT À UN TRAITEMENT PAR PRESSION NÉGATIVE ENDOLUMINAL

(30) Priorität: 10.08.2016 DE 102016114786
(43) Veröffentlichungstag der Anmeldung: 19.06.2019
(73) Patentinhaber: Paul Hartmann AG, 89522 Heidenheim (DE)
(72) Erfinder: GÖRL, Rudolf, 67853 Obernburg (DE); ECKSTEIN, Axel, 89522 Heidenheim an der Brenz (DE)
(86) Internationale Anmeldenummer: PCT/EP2017/070135
(87) Internationale Veröffentlichungsnummer: WO 2018/029229

(56) Entgegenhaltungen:
- EP-A1- 2 851 102
- DE-A1-102012 023 061
- DE-A1-102013 202 849
- DE-T2- 60 030 966
- RUDOLF MENNIGEN ET AL: "Novel treatment options for perforations of the upper gastrointestinal tract: Endoscopic vacuum therapy and over-the-scope clips", WORLD JOURNAL OF GASTROENTEROLOGY, Bd. 20, Nr. 24, 28. Juni 2014 (2014-06-28) , Seiten 7767-7776, XP055410851, CN ISSN: 1007-9327, DOI: 10.3748/wjg.v20.i24.7767

## Beschreibung

Die Erfindung betrifft eine Vorrichtung zur Verwendung bei der Unterdrucktherapie von endoluminalen Wundstellen im Gastrointestinaltrakt.

Unterdrucktherapie von Wunden, im Fachbereich auch bezeichnet als topische negative Druckbehandlung (TNP = topical negative pressure) oder NPWT (negative pressure wound therapy), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt, insbesondere in der initialen Phase der Wundbehandlung, eine effektive Wundreinigung durch Abtransport von Wundexsudat. Als weitere Vorteile werden, insbesondere in der nachfolgenden Granulationsphase, Förderung der Bildung von Granulationsgewebe und Verminderung der Wundödembildung genannt.

Unterdrucktherapie kann bei akuten oder bei chronischen Wunden eingesetzt werden. Hierbei können auch infizierte Wunden erfolgreich behandelt werden. Insbesondere wird die Unterdrucktherapie eingesetzt bei Geschwüren (Ulcus, z.B. Druckgeschwüren, diabetischen Geschwüren, neuropathischen Geschwüren oder venös bedingten Geschwüren), Druckwunden (Dekubitus), traumatischen Wunden, therapieresistenten Wunden, postoperativen Wunden, sondierten Fisteln sowie Hautlappen und Hauttransplantaten. Chronische Wunden sind durch eine verlangsamte oder fehlende Wundheilung gekennzeichnet.

Vorrichtungen zur Unterdrucktherapie von Wunden sind entsprechend im Stand der Technik bekannt. Geeignete Unterdrucktherapiegeräte und Zubehör werden beispielsweise in den Dokumenten WO2011018132, WO2011018133, WO2012156140, WO2012156174, WO2012163617 und WO2013159904 beschrieben. In den Wundraum können beispielsweise die aus den Dokumenten WO2010072309, WO2012022484, WO2011072840, WO2012167942 oder WO2011003521 bekannten Wundauflagen eingebracht werden. Als besonders vorteilhaft in der Unterdruck-Wundbehandlung hat sich nach der Erfahrung des Anmelders eine Wundauflage aus einem offenzelligen Polyester-Polyurethanschaum, die in der EP2515812B1 (aus WO2012022485) näher beschrieben ist, erwiesen.

Geräte zur Unterdrucktherapie von Wunden sind auch kommerziell erhältlich und umfassen kleine, tragbare Geräte, die den Patienten eine gewisse Mobilität ermöglichen bis hin zu stationär zu verwendenden Geräten, etwa in Langzeitpflegeeinrichtungen. Auch für die Unterdrucktherapie geeignetes Zubehör wie beispielsweise Wundauflagen, weitere Verbandkomponenten oder Anschlussmittel sind kommerziell erhältlich.

Ein neues Anwendungsgebiet eröffnet sich für die Unterdrucktherapie bei der Behandlung von Wundstellen im Lumen des Verdauungstraktes. Indikationen für eine Unterdruckapplikation im Gastrointestinaltrakt umfassen Entzündungen, Verätzungen, Anastomose-Insuffizienzstellen und Tumorexzissionsstellen. Chirurgische Anastomosen entstehen nach Resektion von Abschnitten des Verdauungstrakts. Eine gastrointestinale Anastomoseinsuffizienz, also eine mangelnde Dichtheit der chirurgisch geschaffenen Verbindung zwischen Abschnitten des Verdauungstraktes, kann als postoperative Komplikation beispielsweise auf eine entzündungs- oder ischämisch bedingte Nahtinsuffizienz zurückgehen. Bei einem Austritt des Inhalts von Magen oder Darm in eine Körperhöhle drohen lebensgefährliche Entzündungen, beispielsweise eine lebensgefährliche Bauchfellentzündung (Peritonitis) bei einem Austritt von Darminhalt die Bauchhöhle. Weitere mögliche Komplikationen umfassen Abszesse und schlimmstenfalls eine Sepsis.

Zu den bisherigen Behandlungsansätzen beim Auftreten von Anastomoseinsuffizienzen gehören beispielsweise eine weitere chirurgische Intervention, die Platzierung eines Stents oder eine medikamentöse Behandlung. Die Erfolgsaussichten der genannten Therapien sind unbefriedigend.

Deutlich verbesserte Heilungschancen bietet inzwischen eine Behandlungsoption zur Versorgung von Anastomoseinsuffizienzen des Gastrointestinaltraktes, welche auf einer endoluminal gesetzten Schaumdrainage bei gleichzeitiger Applikation von Unterdruck beruht. Diese Behandlungsmethode wird auch als endoluminale Vakuumtherapie (EVT) oder als endoluminale Unterdrucktherapie bezeichnet. Eine Übersicht zu neuartigen Behandlungsverfahren bei Perforationen des oberen Gastrointestinaltraktes findet sich beispielsweise in Menningen R, Senninger N und Laukötter MG (2014) World Journal of Gastroenterology, Vol. 20, No. 24, S. 7767 - 76. Fallberichte zum erfolgreichen Einsatz der endoluminalen Vakuumtherapie bei ösophagalen Perforation werden beispielsweise in Hampe J (2014) Annals of Thoracic Surgery, Vol. 97, No. 3, S. 1029 - 1035 beschrieben.

Anastomoseinsuffizienzen des Gastrointestinaltraktes umfassen kleinere Undichtigkeiten an der Nahtstelle bis hin zu einer ausgeprägten Abzesshöhle. Im Falle von Kavitäten, welche seitlich aus dem Lumen der Verdauungsorgane abzweigen können, wurden Volumina von 40 cm³ bis hin zu 160 cm³ beschrieben, wobei sich die Aussackung auf eine Länge von bis zu 8 cm erstrecken kann. Die Öffnungen der Kavität weisen typischerweise Durchmesser im Bereich von 2 - 4 cm auf.

Bei einer beabsichtigten Behandlung einer endoluminalen Wundstelle mittels Unterdruck muss ein mit einem Absaugschlauch verbundener Fluidsammelkörper geeigneter Größe bereitgestellt und passgenau zur Zielstelle gebracht werden. Ein mit einem Absaugschlauch verbundener Fluidsammelkörper wird im Folgenden auch als "Saugkörper" bezeichnet. Als Fluidsammelkörper wird üblicherweise ein Schwammkörper, welcher insbesondere einen porösen Polymerschaumstoff umfasst, verwendet. Bei der Zielstelle kann es sich im einfacheren Falle um das Innere des Hauptlumens eines Abschnittes des Gastrointestinaltraktes handeln, beispielsweise um die Speiseröhre, um den Dickdarm oder um den Dünndarm.

Eine besondere Herausforderung für den behandelnden Arzt ergibt sich bei der Applikation des Saugkörpers in eine aus dem Hauptlumen abzweigende Aussackung. Eine derartige Aussackung kann insbesondere infolge einer Anastomoseinsuffizienz entstehen. Der Saugkörper kann nur dann in die Kavität eingeführt werden, wenn die Aussackung spiegelbar, d.h. mit einem Endoskop zugänglich ist. Die Größe des Fluidsammelkörpers muss an die Abmessung der Kavität angepasst sein. Die Abmessung der sackartig beschaffenen Kavität wird vor der Applikation des Saugkörpers endoskopisch ermittelt. Nach dem Einführen des Saugkörpers in das zu behandelnde, von der Speiseröhre oder von dem Darm abzweigende Lumen, wird das aus dem Patienten herausragende Ende des Absaugschlauches mit einer Unterdruckquelle verbunden und Unterdruck angelegt. Infolge des Unterdrucks kollabiert die zu behandelnde Kavität teilweise, wobei sich ein enger Kontakt zwischen der Wand der Kavität und dem Fluidsammelkörper einstellt. Der Kontakt zwischen Fluidsammelkörper und der Wand des Lumens stimuliert die Bildung von Granulationsgewebe. Wundfluide werden über den Fluidsammelkörper und den Absaugschlauch abgesaugt, so dass die Wunde permanent gesäubert wird und Infekte unter Kontrolle gehalten werden. Des Weiteren führt die Unterdruckapplikation zu einer kontinuierlichen Konstriktion der Ausstülpung. Der Saugkörper muss nach einigen Tagen kontinuierlicher Unterdruckapplikation immer wieder ersetzt werden, wobei die Größe des Fluidsammelkörpers bei fortschreitender Verkleinerung der Kavität schrittweise reduziert wird. Die Unterdruckbehandlung kann beendet werden, wenn die Aussackung weitestgehend zurückgegangen ist.

Im Falle einer Behandlung einer Anastomoseinsuffizienz ohne Aussackung wird die Therapie beendet, wenn die Leckage geschlossen ist.

Die Erfolgschancen des endoskopischen Eingriffs hängen wesentlich von der Auswahl eines passend dimensionierten Fluidsammelkörpers sowie von einer optimalen Platzierung des Saugkörpers in der Wundhöhle ab. Um den Saugkörper in die Kavität einzuführen, sind aus der medizinischen Praxis unterschiedliche Ansätze bekannt.

Die EP1572286-B1 beschreibt ein Behandlungssystem zur endoskopischen Wundversorgung, welches insbesondere zur Behandlung perianastomotischer Abszesse vorgesehen ist. Das Behandlungssystem umfasst einen Fluidsammelkörper, einen mit dem Fluidsammelkörper verbundenen Absaugschlauch sowie eine Einführhilfe (Inserter). Die Einführhilfe besteht aus einem dickeren Schlauch (Outer Sleeve bzw. Overtube) und aus einem dünneren Schlauch (Inner Sleeve bzw. Pusher). Der Overtube wird über ein bewegliches Endoskop in die Wundhöhle geschoben. Nach dem Entfernen des Endoskops wird der Fluidsammelkörper durch den Overtube geschoben und mithilfe des Pusher positioniert bzw. freigesetzt. Ein derartiges endoskopisches Behandlungssystem ist unter der Bezeichnung Endo-SPONGE^{®} für den unteren Gastrointestinaltrakt und unter der Bezeichnung Eso-SPONGE^{®} für den oberen Gastrointestinaltrakt im Handel erhältlich (Firma B. Braun Melsungen AG, Deutschland). Ein Einführsystem mit Positionierungshülse und Führungshülse ist beispielsweise auch aus der DE102009043472 bekannt.

Eine alternatives Verfahren zur präzisen Platzierung des Saugkörpers an der gewünschten Wundstelle beruht darauf, den am Saugkörper vorhandenen Schwamm mit einer am Ende eines endoskopischen Instrumentes angebrachten Zange zu ergreifen und dann mittels des Endoskops in die Wundtasche zu ziehen. Gemäß einer in der Praxis üblichen Vorgehensweise wird hierbei unmittelbar vor der endoskopischen Behandlung durch den behandelnden Arzt unter Einsatz chirurgischen Nahtmaterials am Wundschwamm eine Fadenschlaufe angebracht. Der Faden wird hierbei mit dem Schwamm und normalerweise auch mit dem Absaugschlauch vernäht, um ein Ausreißen des Schwamms zu vermeiden. Die EP2769744 beschreibt ein Medizinprodukt zur Drainage pathologischer Flüssigkeitsansammlungen, welches einen Fluidsammelkörper und einen mit dem Fluidsammelkörper verbundenen Absaugschlauch umfasst. Innerhalb des Absaugschlauches ist ein Faden vorhanden, welcher schlaufenförmig aus dem bei der Behandlung zur Wundhöhle hin zeigenden Ende des Absaugschlauches heraushängt. Die Fadenschlaufe dient als Fassschlaufe zur Positionierung des Saugkörpers mittels eines Endoskops.

Der vorliegenden Erfindung lag die Aufgabe zugrunde, eine Vorrichtung bereit zu stellen, um die endoluminale Unterdrucktherapie weiter zu verbessern. Insbesondere sollte die Handhabung der endoskopischen Komponenten vereinfacht werden, die Dauer des endoskopischen Eingriffes verkürzt und die Anwendungssicherheit erhöht werden.

Die Erfindung löst die oben genannten Aufgaben durch Bereitstellung einer Vorrichtung zur Verwendung bei der Unterdrucktherapie von endoluminalen Wundstellen im Bereich des Gastrointestinaltraktes gemäß Anspruch 1.

Die erfindungsgemäße Vorrichtung umfasst einen Schwammkörper, welcher zum Auskleiden einer Wundhöhle und zur Aufnahme von Fluiden aus dieser Wundhöhle vorgesehen ist. Bei den aus der Wundhöhle aufzunehmenden Fluiden handelt es sich insbesondere um flüssige und zähflüssige Substanzen, beispielsweise um Blut, Schleim, Eiter, aber auch um von außen während der Behandlung zugeführte Flüssigkeiten. Bei dem Schwammkörper handelt es sich also um einen Fluidsammelkörper. Der Schwammkörper umfasst vorzugsweise einen polymeren Schaumstoff, insbesondere einen offenzelligen Polymerschaum aus Polyurethan oder Polyvinylalkohol. Der Schwammkörper besteht vorzugsweise vollständig aus einem polymeren Schaumstoff. Besonders bevorzugt wird ein Polymerschaumstoff aus einem Polyester-Polyurethan eingesetzt, wie beispielsweise in der EP2515812B1 beschrieben. Weiterhin umfasst die erfindungsgemäße Vorrichtung einen mit dem Schwammkörper unlösbar verbundenen Absaugschlauch. Dieser dient zur Beaufschlagung der Wundstelle mit Unterdruck und zum Abtransport der von dem Fluidsammelkörper aufgenommenen Substanzen. Der Absaugschlauch fungiert somit als medizinischer Drainageschlauch. Der Schwammkörper umschließt einen Abschnitt des Absaugschlauches zumindest teilweise, so dass der Absaugschlauch also in dem genannten Abschnitt innerhalb des Schwammkörpers verläuft. Der Schwammkörper ist mit dem Absaugschlauch an seinem wundseitigen Ende oder nahe seines wundseitigen Endes verbunden. Zumindest an dem von dem Schwammkörper umschlossenen Abschnitt des Absaugschlauchs sind eine oder mehrere Öffnungen vorhanden. Durch die Öffnungen kann die Wundhöhle mit Unterdruck beaufschlagt werden und es können Fluide abgesaugt werden. Da die Öffnungen an dem innerhalb des Schwammkörpers verlaufenden Abschnitt des Absaugschlauchs vorhanden sind, werden die Fluide durch den Schwammkörper hindurch zum Absaugschlauch gesaugt. Der Schwammkörper, welcher bei der Behandlung die Wundhöhle möglichst vollständig auskleiden soll, dient somit auch als Fluidsammelkörper.

Erfindungsgemäß ist die Vorrichtung dadurch gekennzeichnet, dass an der Vorrichtung eine bereits herstellerseitig angebrachte Fassschlaufe vorhanden ist. Die Fassschlaufe ist hierbei entweder aus einem Abschnitt des Schwammkörpers, oder aus einem Abschnitt des Absaugschlauchs geformt, wobei die Fassschlaufe durch Stanzen einer Öffnung in den Schwammkörper aus einem Abschnitt des Schwammkörpers geformt ist, oder durch Stanzen einer Öffnung in den Absaugschlauch aus einem Abschnitt des Absaugschlauchs geformt ist. Die Fassschlaufe dient zum Ergreifen der Vorrichtung und dann zum Führen des Schwammkörpers zur Wundstelle hin, wobei das Ergreifen und das zum Zielort hin translozieren üblicherweise durch ein endoskopisches Instrument unter Kamerakontrolle erfolgt. Hierzu weist das endoskopische Instrument an seinem Ende eine miniaturisierte Greifzange auf, welche die Fassschlaufe fest, jedoch reversibel packen und zum Zielort mitziehen kann. Es geht also bei der vorliegenden Erfindung insbesondere um eine bereits herstellerseitig an der Vorrichtung zur endoluminalen Unterdrucktherapie vorhandene Fassschlaufe, die mit einem medizinischen Instrument ergriffen werden kann. Unter dem Begriff "Fassschlaufe" wird im Zusammenhang mit der vorliegenden Erfindung allgemein eine griffförmige Struktur verstanden, welche geeignet ist, von einem an einem endoskopischen Instrument vorhandene Greifmechanismus, insbesondere von einer Greifzange, reversibel festgehalten zu werden. Die Fassschlaufe soll hinsichtlich ihrer Abmessungen und ihrer Form an die an dem endoskopischen Instrument vorhandene Greifzange angepasst werden.

Im Gegensatz zu bisher üblichen Verfahren, bei denen der behandelnde Arzt einen Faden an den Saugkörper angebracht hat, ist die erfindungsgemäß vorgestellte Methode schneller, zuverlässiger und sicherer. Der Arzt kann sich vollständig auf die bevorstehende Operation konzentrieren und muss sich nicht mit der Vorbereitung des Wundkörpers befassen. Die Fassschlaufe ist stabil und kann optimal auf das endoskopische Instrument abgestimmt werden. Ein unbeabsichtigtes Abfallen des Schwammes von dem endoskopischen Instrument wird weitestgehend vermieden. Die Fassschlaufe ermöglicht eine präzise Applikation des Schwammkörpers mittels eines flexiblen Endoskops. Ebenso gewährleistet eine auf das endoskopische Instrument abgestimmt Fassschlaufe eine unproblematische Freisetzung des Saugkörpers am Zielort: Im Gegensatz zu herkömmlichen Methoden wird die Gefahr reduziert, dass sich Fadenschlingen an der Greifzange des endoskopischen Instrumentes verheddern und so das Abkoppeln des Saugkörpers vom Instrument beeinträchtigen.

Gemäß einer ersten Variante der Erfindung wird die Fassschlaufe herstellerseitig aus einem Abschnitt des Absaugschlauchs geformt. Hierbei wird die Fassschlaufe durch Stanzen einer Öffnung in den Absaugschlauch geformt. Die eingestanzte Öffnung kann in Längsrichtung des Absaugschlauches einen Durchmesser von 2 mm bis 200 mm aufweisen. Vorzugsweise beträgt ihre Länge zwischen 3 mm und 25 mm, wobei die Länge der Öffnung an die Abmessungen der Greifzange des endoskopischen Instrumentes abgestimmt ist. Der maximale Durchmesser der Stanzöffnung in Querrichtung hängt von der Dicke des Absaugschlauches ab. Der Durchmesser der eingebrachten Öffnung in Querrichtung darf den Durchmesser des Lumens des Absaugschlauches nicht überschreiten, um die Stabilität der Fassschlaufe zu gewährleisten. So darf beispielsweise bei einem Absaugschlauch mit einem äußeren Durchmesser von 5 mm und einem inneren Durchmesser des Lumens von 3 mm der Durchmesser der Stanzöffnung in Querrichtung 4 mm nicht überschreiten. Eine geeignete Abmessung für eine Fassschlaufe an einem medizinischen Absaugschlauch mit kreisförmigen Querschnitt und mit einem äußeren Durchmesser von 5 mm und einem Durchmesser des Lumens von 3 mm ist beispielsweise 5 mm in Längsrichtung und 4 mm in Querrichtung. Eine derartige Fassschlaufe lässt sich gut greifen und ist stabil. Das Einbringen der Stanzöffnung erfolgt nach üblichen Stanzverfahren und kann bei Raumtemperatur oder nach Erhitzen des Schlauches erfolgen.

Der Schwammkörper und der Absaugschlauch des Saugkörpers sollten derart miteinander verbunden sein, dass die Fassschlaufe vollständig oder zumindest teilweise aus dem Schwammkörper herausragt, so dass ein Ergreifen der Fassschlaufe durch das endoskopische Instrument ungehindert gewährleistet ist.

Gemäß einer nicht erfindungsgemäßen Ausführung einer Vorrichtung zur Verwendung bei der Unterdrucktherapie von endoluminalen Wundstellen im Bereich des Gastrointestinaltraktes wird die Fassschlaufe herstellerseitig aus einem mit dem Absaugschlauch unlösbar verbundenem Materialabschnitt geformt. Hierzu formt der Hersteller beispielsweise aus einem Kunststoffmaterial zunächst eine zum Greifen durch ein endoskopisches Instrument angepasste Fassschlaufe. Der eine Fassschlaufe umfassende Materialabschnitt wird dann unlösbar mit dem Ende des Absaugschlauches verbunden. Eine unlösbare Verbindung kann beispielsweise durch Verpressen, durch Verkleben oder durch Vulkanisieren hergestellt werden. In der Praxis kann der Materialabschnitt neben der Fassschlaufe einen zapfenartigen Anteil umfassen, welcher zur unlösbaren Verbindung mit dem Absaugschlauch vorgesehen ist. Der zapfenartige Anteil des Materialabschnittes wird in den Absaugschlauch eingeschoben und mit dem Schlauch verklebt. Bei der hier beschriebenen Ausführungsform ist ebenso wie bei der vorangehend beschriebenen Ausführungsform darauf zu achten, dass die Fassschlaufe zumindest teilweise aus dem Schwammkörper herausragt, so dass ein Ergreifen der Fassschlaufe durch das endoskopische Instrument ungehindert gewährleistet ist.

Nach dieser nicht erfindungsgemäßen Ausführung besteht der Materialabschnitt aus einem Faden, wobei der Faden beispielsweise aus einem textilen Material oder aus einem flexiblen Kunststoffmaterial hergestellt werden kann. Der Faden wird dann an der Außenseite des Absaugschlauches unlösbar befestigt, insbesondere durch Verkleben. Um eine besonders stabile Verbindung zwischen dem Absaugschlauch und dem Faden zu gewährleisten, sollte der Faden vorzugsweise aus einem Kunststoffmaterial bestehen.

Vorzugsweise ragt die Fassschlaufe vollständig aus dem Schwammkörper heraus.

Gemäß einer zweiten Variante der vorliegenden Erfindung wird die Fassschlaufe herstellerseitig durch Stanzen einer Öffnung in den Schwammkörper, insbesondere durch Stanzen einer Öffnung in einen Polymerschaumstoff, geformt. Das Stanzen erfolgt vorzugsweise unter Hitzeeinwirkung. Um die Stabilität einer durch Stanzen aus dem Polymerschaumstoff geformten Fassschlaufe zu erhöhen, kann der Polymerschaumstoff nach dem Stanzen im Bereich der Fassschlaufe gehärtet werden. Eine Härtung des die Fassschlaufe umgebenden Materials ist beispielsweise durch Verpressen (insbesondere durch Verpresssen unter Hitzeeinwirkung) oder durch chemische Behandlung mit einem aushärtbaren Polymer möglich. Zur Stabilisierung der durch Stanzen aus dem Schaum erzeugten Fassschlaufe können beispielsweise Polymergemische auf der Basis von Silikon, Polyurethan, Polyacetate, Polycarbonat, Polyethylen, Weich- Polyvinylchlorid, Polyvinylalkohol oder thermoplastische Polymere verwendet werden. Die Modifikation des die Fassschlaufe umgebenden Materials sollte idealerweise zur Auflösung der Stege des Polymerschaumstoffs führen, so dass ein massives (nicht-porösen) Kunststoffmaterial gebildet wird, welches die Fassschlaufe umläuft. Dies hat den Vorteil, dass sich die Greifzange des endoskopischen Instrumentes beim Loslassen des Saugkörpers dann nicht mehr in den Stegen des Polymerschaumstoffs verfangen kann.

Bei den zwei vorgenannten Varianten der erfindungsgemäßen Vorrichtung weist die Fassschlaufe vorzugsweise eine Länge zwischen 3 mm und 25 mm, besonders bevorzugt zwischen 3 mm und 15 mm, auf. Gemäß einer weiteren besonders bevorzugten Ausführungsform beträgt die Länge zwischen 10 mm und 25 mm. In der Praxis hat sich insbesondere eine Länge der Fassschlaufe von 12 mm (+/-2 mm) als besonders vorteilhaft erwiesen.

Nach einem weiteren nicht erfindungsgemäßen Gedanken kann ein Faden herstellerseitig an einer Abschnürung des Schwammkörpers befestigt wird. Der Faden dient dann als Fassschlaufe. Um den Faden an der Abschnürung anzubringen, kann der Faden ein- oder mehrmals um die Abschnürung herumgewickelt und durch Verknoten befestigt werden. Die Abschnürung sollte hierbei vorzugsweise an dem wundnahen Ende des Schwammkörpers vorhanden sein. Der Faden kann beispielsweise aus einem textilen Material oder aus einem flexiblen Kunststoffmaterial bestehen. Eine nach diesem weitere Erfindungsgedanken ausgestaltete Vorrichtung zur Verwendung bei der Unterdrucktherapie von endoluminalen Wundstellen im Bereich des Gastrointestinaltraktes umfasst einen Schwammkörper und einen mit dem Schwammkörper unlösbar verbundenen Absaugschlauch. Der Schwammkörper umschließt einen Abschnitt des Absaugschlauches zumindest teilweise. Zumindest an dem von dem Schwammkörper umschlossenen Abschnitt des Absaugschlauches sind Öffnungen vorhanden. Der Schwammkörper weist insbesondere an seinem wundnahen Ende eine Abschnürung auf, an welcher ein Faden als Fassschlaufe befestigt ist. Die Länge des Fadens liegt vorzugsweise im Bereich 20 mm - 100 mm.

Der an dem Absaugschlauch unlösbar angebrachte Schwammkörper umfasst vorzugsweise einen offenzelligen Polymerschaum, insbesondere einen retikulierten Polymerschaumstoff. Bei dem Polymer handelt es sich vorzugsweise um Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan, Kollagen oder um eine Mischung aus den genannten Polymeren.

In einer besonders bevorzugten Ausführungsform umfasst der Schwammkörper einen offenzelliger Polyurethanschaumstoff, der erhältlich ist durch Umsetzung einer Mischung umfassend die Komponenten (i) Polyisocyanat, (ii) Polyol, bevorzugt Polyesterpolyol, (iii) Treibmittel und (iv) Katalysator.

Als Schaumstoffe werden üblicherweise Werkstoffe mit über die gesamte Masse verteilten Zellen (offen, geschlossen oder beides) verstanden. Somit weisen solche Werkstoffe üblicherweise eine Rohdichte (gemäß DIN EN ISO 845) auf, die niedriger ist als die Dichte der Gerüstsubstanz. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt. Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf. Bei dem offenzelligen Schaumstoff kann es sich um einen retikulierten oder um einen nichtretikulierten Schaumstoff handeln. Unter einem retikulierten Schaumstoff wird ein Schaumstoff verstanden, der im Wesentlichen nur Zellstege aufweist. Bei einem retikulierten Schaumstoff sind die Zellwände somit im Wesentlichen entfernt. Die Retikulierung wird üblicherweise in einer Druckkammer, z.B. einer Stahlkammer durchgeführt. Nach Einbringen des Schaumstoffs in die Stahlkammer wird die Luft abgesaugt (bevorzugt zu 50 bis 100 Gew.-%, mehr bevorzugt zu 70 bis 99 Gew.-%) und durch ein Brenngasgemisch, bevorzugt durch ein Gemisch enthaltend Wasserstoff und Sauerstoff, insbesondere im molaren Verhältnis von 2 : 1 ersetzt. Bei der Zündung des Gasgemisches zerreißen die Zellhäute durch die entstehende Hitze- und Druckwelle. Gegebenenfalls erfolgt auch ein zumindest teilweises Aufschmelzen der Zellstege, so dass diese verstärkt werden.

Es hat sich ferner gezeigt, dass vorstehend genannte Aufgaben unerwartet vorteilhaft gelöst werden können, wenn der im Schwammkörper vorhandene Schaumstoff eine spezielle Luftdurchlässigkeit aufweist. In einer bevorzugten Ausführungsform weist der Schaumstoff eine Luftdurchlässigkeit von 1000 bis 8000 l/(m²sec), mehr bevorzugt von 1500 bis 6000 l/(m²sec), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) auf.

Hierbei weist der im Schwammkörper vorhandene Schaumstoff insbesondere eine Bruchdehnung von 100 % bis 400 %, mehr bevorzugt von 120 % bis 300 %, noch mehr bevorzugt von 150 % bis 200 %, gemessen gemäß DIN 53571, auf.

Gemäß einer weiteren bevorzugten Ausführungsform der Erfindung weist der im Schwammkörper vorhandene Schaumstoff eine Zellzahl (= Anzahl der Poren entlang einer Geraden pro laufendem Inch) von mindestens 5 und höchstens 400 Zellen pro Inch auf. Gemäß einer alternativen, gleichfalls sehr vorteilhaften Ausführungsform, beträgt die Zellzahl des im Schwammkörper vorhandenen Schaumstoffs mindestens 10 und höchstens 200 Zellen pro Inch. Die Zellzahl wird bevorzugt mikroskopisch bestimmt.

Der Schwammkörper kann auch zwei oder mehr konzentrisch vorliegende Lagen, insbesondere zwei oder mehr konzentrisch vorliegende Lagen aus Schaumstoff, umfassen. Hierbei ist es besonders vorteilhaft, wenn die äußerste Lage eine Zellzahl zwischen 5 und 200 Zellen pro Inch, vorzugsweise eine Zellzahl zwischen 6 und 100 Zellen pro Inch, besonders bevorzugt eine Zellzahl zwischen 8 und 30 Zellen pro Inch aufweist. Gleichzeitig sollte eine weiter innen vorhandene zweite Lage vorzugsweise zwischen 10 und 400 Zellen pro Inch, besonders bevorzugt zwischen 15 und 200 Zellen pro Inch und insbesondere zwischen 40 und 60 Zellen pro Inch aufweisen. Bei einem derartigen Schwammkörper, welcher zwei oder mehr konzentrisch vorliegende Lagen aus Schaumstoffen mit einer unterschiedlichen Zellzahl umfasst, kann eine besonders effektive Ableitung von Fluiden im Inneren des Schwammkörpers vorteilhaft mit einer besonders vorteilhaft ausgestalteten Wundkontaktfläche kombiniert werden.

Gemäß einer weiteren vorteilhaften Ausführungsform ist vorgesehen, dass die Rohdichte des Schaumstoffs zwischen 10 und 350 kg/m³ beträgt, gemessen gemäß DIN EN ISO 845. Besonders bevorzugt beträgt die Rohdichte 15 bis 65 kg/m³, insbesondere 20 bis 45 kg/m³. Die Stauchhärte des für den Schwammkörper vorgesehenen Schaumstoffs beträgt vorzugsweise mindestens 1,5 kPa und höchstens 22,5 kPa, gemessen nach ISO 3386-1.

Der von der Vorrichtung umfasste Schwammkörper kann mit einem Zusatz und/oder Hilfsstoff beschichtet oder imprägniert sein. Bei dem Zusatz oder der Imprägnierung kann es sich beispielsweise um eine die Wundheilung fördernde Salbengrundlage handeln, wobei die Salbengrundlage vorzugsweise zusätzlich einen Quellstoff umfasst. Exemplarisch wird auf die WO2012/167943 verwiesen, welche im Rahmen der Erfindung geeignete

Salbengrundlagen beschreibt. Eine im Rahmen der Erfindung besonders geeignete Salbengrundlage, welche zusätzlich einen Quellstoff umfasst, ist in Beispiel 1 der Patentanmeldung WO2012/167943 beschrieben.

Der von der Vorrichtung umfasste Schwammkörper kann alternativ oder zusätzlich zu der vorstehend beschriebenen Salbengrundlage eine antimikrobiell wirksame Substanz umfassen, beispielsweise Polyhexanid. Bei der antimikrobiell wirksamen Substanz kann es sich auch um ein Antibiotikum handeln, beispielsweise um Oxytetracyclin oder um eine Kombination von Bacitracin und Neomycin. Gegebenenfalls kann der Zusatz oder die Imprägnierung zusätzlich zum Antibiotikum oder alternativ eine entzündungshemmende Substanz umfassen, beispielsweise Hydrocortison.

Gemäß einer besonders bevorzugten Ausführungsform umfasst die antimikrobiell wirksame Substanz Silber oder eine Silber-Verbindung.

Die Schwammkörper kann Silber in Form von Silberionen oder in Form von atomarem Silber enthalten. Bevorzugt ist Silber in Form einer Silberbeschichtung auf der Oberfläche des Schwammkörpers aufgebracht. Alternativ kann das Silber innerhalb des Schwammkörpers verteilt sein. Beispielsweise kann bei offenzelligen Schaumstoffen Silber bereits in die härtbare Zusammensetzung mit eingebracht werden. Bevorzugt enthält der im Schwammkörper vorhandene Schaumstoff 0,000001 bis 0,1 Gew.-% Silber, mehr bevorzugt 0,0001 bis 0,01 Gew.-% Silber, bezogen auf das Gesamtgewicht des jeweiligen Schaumstoffs. Besonders bevorzugt enthält der im Schwammkörper vorhandene Schaumstoff 0,0001 bis 0,01 Gew.-% Silber, bezogen auf das Gesamtgewicht des Schaumstoffs.

Des Weiteren kann der Schwammkörper einen Partikel-förmigen Zusatz umfassen, wobei es sich bei dem Partikel-förmigen Zusatz insbesondere um ein quellfähiges Polymer handelt. Gemäß einer bevorzugten Ausführungsform umfasst Schwammkörper Partikel aus einem superabsorbierenden Polymer (SAP).

Hinsichtlich seiner äußeren Form ist der Schwammkörper im Wesentlichen zylinderförmig ausgebildet, wobei zusätzlich ein zum wundnahen Ende oder zum Absaugschlauch hin abgeschrägt oder gebogen verlaufender Abschnitt vorhanden sein kann. Der Schwammkörper weist eine Länge von 3 bis 10 cm sowie einen Durchmesser von 1 bis 5 cm auf. Bei dem Zylinder handelt es sich insbesondere um einen senkrechten Kreiszylinder. Der hier verwendete Begriff "im Wesentlichen zylinderförmig" umfasst auch solche senkrechten Kreiszylinder, welche eine oder zwei abgerundete oder abgeschrägte Kanten aufweisen.

Der Schwammkörper ist an demjenigen Ende des Absaugschlauches mit dem Absaugschlauch unlösbar verbunden, welches im Gebrauch der Vorrichtung in den Wundbereich eingeschoben wird.

Der Absaugschlauch umfasst bevorzugt einen für medizinische Zwecke geeigneten Kunststoff, insbesondere Silikon, Polyacetat, Polycarbonat, Polyethylen, Polyurethan, Weich-Polyvinylchlorid (PVC, welches durch einen Weichmacher modifiziert wurde, beispielsweise durch Tri-2-ethylhexyl Trimellitate - "TOTM"), Polyvinylalkohol, Thermoplastische Polymere oder eine Mischung daraus.

Bei der erfindungsgemäßen Vorrichtung ist vorzugsweise am Absaugschlauch ein Konnektor zum Verbinden des Absaugschlauches mit einer Unterdruckquelle vorhanden. Bei dem Konnektor handelt es sich insbesondere um einen Schnelltrennverbinder. Der Konnektor ist an demjenigen Ende des Absaugschlauches angebracht, welches bei Gebrauch der Vorrichtung außerhalb des Patienten vorhanden ist.

Bei der Unterdruckquelle handelt es sich insbesondere eine Saugflasche oder eine elektrisch antreibbare Unterdruckquelle. Eine im Zusammenhang mit der vorliegenden Erfindung besonders geeignete elektrisch antreibbare Unterdruckquelle ist kommerziell unter der Bezeichnung VivanoTec^{®} erhältlich (Paul Hartmann AG, Heidenheim).

Die Erfindung umfasst gleichermaßen ein Verfahren zur Herstellung einer Vorrichtung zur Verwendung bei der Unterdrucktherapie von endoluminalen Wundstellen im Bereich des Gastrointestinaltraktes.

Das Verfahren umfasst die nachfolgenden Schritte, welche allesamt herstellerseitig durchgeführt werden:
i) Bereitstellen eines für medizinische Zwecke geeigneten Schwammkörpers, insbesondere eines Schwammkörpers aus einem Polymerkunststoff.
ii) Bereitstellen eines für medizinische Zwecke geeigneten Absaugschlauches.
iii) Herstellen einer unlösbaren Verbindung zwischen dem Schwammkörper und dem Absaugschlauch.
iv) Formen einer Fassschlaufe. Die Fassschlaufe kann hierbei gemäß einer ersten Variante des Verfahrens aus einem Abschnitt des Absaugschlauchs geformt werden. Die Fassschlaufe kann gemäß einer zweiten Variante des Verfahrens aus einem Abschnitt des Schwammkörpers geformt werden.
v) Das Verfahren umfasst optional ein Sterilisieren und gebrauchsfertiges Konfektionieren der Vorrichtung.

Die Reihenfolge der oben genannten Schritte iii) und iv) kann im Zusammenhang mit der Erfindung beliebig gewählt werden. Es ist möglich zunächst eine unlösbare Verbindung zwischen dem Schwammkörper und dem Absaugschlauch herzustellen und erst danach eine Fassschlaufe zu formen. In den meisten Fällen wird es jedoch praktischer sein, zunächst die an dem Absaugschlauch oder an dem Schwammkörper vorhandene Fassschlaufe zu formen und dann den Absaugschlauch mit dem Wundschwamm zu verbinden.

Gemäß der ersten Variante des Verfahrens wird die Fassschlaufe durch Stanzen einer Öffnung in den Absaugschlauch geformt. Hierzu kann der Absaugschlauch vor dem Stanzvorgang erhitzt werden. Das Einbringen der später bei Gebrauch als Fassschlaufe dienenden Öffnung erfolgt an demjenigen Ende des Absaugschlauches, welches während der Wundbehandlung in die zu behandelnde Wundhöhle eingeführt wird.

Die vorliegende Erfindung betrifft gleichfalls ein Kit zur Verwendung bei der Unterdrucktherapie von endoluminalen Wundstellen im Bereich des Gastrointestinaltraktes. Das Kit umfasst folgende Bestandteile:
- eine Vorrichtung nach einer der vorstehend beschriebenen Varianten und Ausführungsformen, wobei sämtliche Bestandteile der Vorrichtung steril abgepackt vorliegen
- ein Gleitgel zum Einführen des Schwammkörpers in ein Lumen des Gastrointestinaltraktes
- optional einen Konnektor zum Verbinden des Absaugschlauches mit einer Unterdruckquelle, wobei der Konnektor vorzugsweise steril abgepackt vorliegt.

Die erfindungsgemäße Vorrichtung kann besonders vorteilhaft zur Unterdruckbehandlung im Gastrointestinaltrakt eingesetzt werden. Sie eignet sich jedoch gleichermaßen zur Verwendung bei der Unterdrucktherapie von ausgedehnten Wundhöhlen außerhalb des Gastrointestinaltraktes.

### Figuren

- Figur 1: Rückbildung einer Abszesshöhle während der Unterdruckbehandlung
- Figur 2 a/b: Erste Variante der erfindungsgemäßen Vorrichtung
- Figur 3 a/b: Zweite Variante der erfindungsgemäßen Vorrichtung
- Figur 4 a/b: Dritte Variante der erfindungsgemäßen Vorrichtung
- Figur 5: Weitere Ausführungsform der zweiten Variante der erfindungsgemäßen Vorrichtung
- Figur 6: Saugkörper mit Abschnürung
- Figur 7 a/b: An einem endoskopischen Instrument befestigter Saugkörper

### Bezugszeichen

- 1: Absaugschlauch
- 2: Schwammkörper
- 3: Fassschlaufe
- 4: Perforation
- 5: Materialabschnitt
- 6: Zapfen
- 7: Abschnitt des Schwammkörpers, welcher die Fassschlaufe enthält
- 8: Übergangsbereich zwischen Abschnitt 7 und dem Schwammkörper
- 11: Speiseröhre mit seitlich abzweigender Kavität
- 12: Kavität
- 13: Anastomose
- 14: Speiseröhre
- 10, 20, 30, 40, 50, 60, 70: Saugkörper
- 51: Kleberauftrag
- 61: Endoskop
- 62: Arbeitskanal
- 63: Mikromechanischer Greifer
- 64: Zange
- 65: Abschnürung

### Figuren 1 a - e

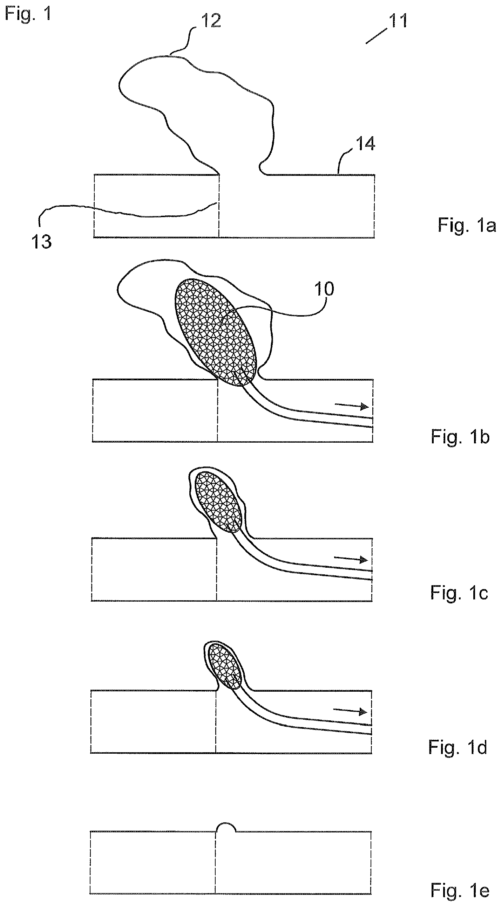

Figuren 1 a - e zeigen einen durch Unterdruckbehandlung unterstützten Heilungsverlauf bei einer aus einer Speiseröhre (14) abzweigenden Kavität (12) in schematischer Darstellung. Die in Figur 1 gezeigte Unterdruckbehandlung einer endoluminalen Kavität ist aus dem Stand der Technik bekannt. Eine aus der Speiseröhre seitlich abzweigende Kavität (12), wie in Figur 1 a dargestellt, kann unter anderem infolge einer chirurgisch erzeugten Anastomose (13) entstehen (beispielsweise nach einer Tumorexzision). Vor der Unterdruckbehandlung wird die Kavität (12) endoskopisch gespiegelt (nicht dargestellt), um ihre Abmessung zu ermitteln. Nachdem ein Fluidsammelkörper (10) geeigneter Größe in die Wundhöhle (12) eingebracht ist, kann über den Absaugschlauch (1) Unterdruck angelegt werden (Figur 1b). Die Unterdruckapplikation bewirkt eine Ableitung von Wundsekreten und unterstützt die Kontraktion der Kavität (12), wie in Figuren 1 b - 1 d angedeutet. Nach einer permanenten Unterdruckbehandlung von einigen Tagen bis zu mehreren Wochen ist die Kavität bereits so weit geschrumpft, dass der Fluidsammelkörper aus den Wundbereich entfernt werden kann (Figur 1 e).

### Figuren 2 a/b

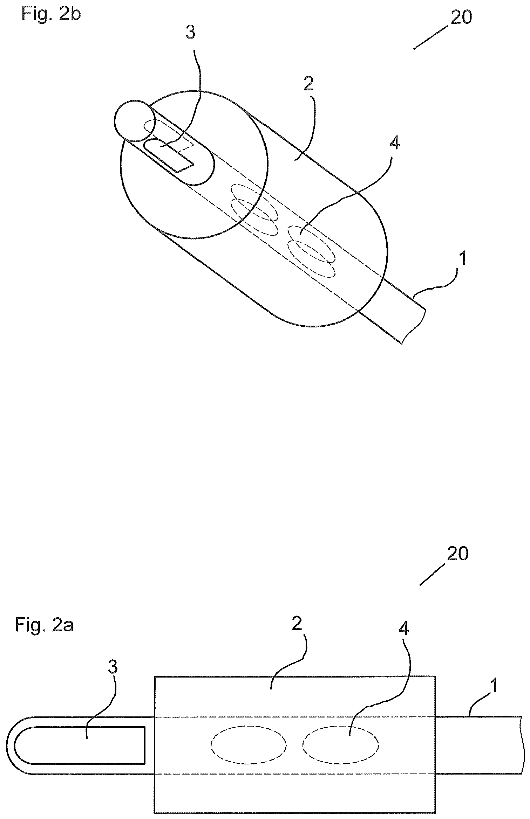

Gemäß der in den Figuren 2 a/b schematisch gezeigten ersten Variante des erfindungsgemäßen Saugkörpers (20) wird eine Fassschlaufe (3) herstellerseitig durch einen Stanzvorgang direkt aus dem Absaugschlauch (1) hergestellt. In demselben Stanzvorgang, in dem die Fassschlaufe (3) geformt wird, können gleichzeitig weitere Öffnungen (4) in den Absaugschlauch (1) eingebracht werden. Diese zusätzlichen Öffnungen (4) werden an dem von dem Schwammkörper (2) umschlossen Abschnitt des Absaugschlauches (1) eingebracht Die Öffnungen (4) sind zur Applikation des Unterdrucks und zum Ansaugen von Fluiden aus dem Wundraum erforderlich. Die zusätzlichen Öffnungen (4), falls vorhanden, können wie in Figur 2 b angedeutet in dem Schlauch gegenüberliegend vorliegen. Sie können auch entlang der Schlauchachse zueinander versetzt vorliegen (nicht dargestellt).

Die Fassschlaufe (3) ragt bei dem dargestellten Beispiel vollständig aus dem Schwammkörper (2) heraus. Da die Fassschlaufe (3) bei dem in den Figuren 2 a/b gezeigten Beispiel mit dem Lumen des Absaugschlauches (1) in einem Kontinuum steht, entsteht während der Behandlung auch ein Sog über die Fassschlaufe (3). Dieser Effekt hat sich als vorteilhaft erwiesen, da die Ableitung zäher oder partikelhaltiger Sekrete hierdurch verbessert wird. Grundsätzlich wäre es jedoch auch möglich, das Lumen des Absaugschlauches (1) gegenüber der Fassschlaufe (3) zu isolieren, so dass der Unterdruck ausschließlich über den Schwammkörper mit dem Wundraum kommunizieren kann (in Figur 2 a/b nicht dargestellt).

Bei dem in den Figuren 2 a/b gezeigten Beispiel wurde die Fassschlaufe (3) in einen Schlauch gestanzt, dessen Ende einen abgerundeten und geschlossenen Endabschnitt aufweist. Obgleich es grundsätzlich möglich wäre, die Fassschlaufe (3) in einen Schlauch einzubringen, dessen Endabschnitt offen vorliegt und eine glatte Schnittfläche aufweist (in Figuren 2 a/b nicht dargestellt), hat sich ein abgerundeter und geschlossener Abschluss des Absaugschlauches als vorteilhaft erwiesen, da hierdurch eine besonders schonende Einbringung des Saugkörpers (20) in den Wundraum erleichtert wird.

Lediglich beispielhaft wird im Folgenden eine mögliche Dimensionierung für einen Schwammkörper (2), einen Absaugschlauch (1) und die aus dem Absaugschlauch (1) geformte Fassschlaufe (3) angegeben:

| | |
|---|---|
| Außendurchmesser des Absaugschlauchs: | 4,6 mm |
| Wandstärke des Absaugschlauchs: | 1,0 mm |
| Durchmesser des Lumens des Absaugschlauches: | 2,6 mm |
| Länge der Fassschlaufe: | 12 mm |
| Seitliche Ausdehnung der Fassschlaufe: | 2,0 mm |

Der in Figuren 2 a/b beispielhaft dargestellte Absaugschlauch (1) ist aus Weich-PVC.

Der Schwammkörper (2) kann beispielsweise einen hydrophoben, retikulierten Polyester-Polyurethan-Schaumstoff umfassen, mit einer Rohdichte gemäß ISO 845 von 26,0 kg/m³, mit einer Stauchhärte gemäß DIN EN ISO 3386-1 von 3,4 kPa, mit einer Bruchdehnung gemäß DIN 53571 von 290 % und mit einer Zellzahl (mikroskopisch bestimmt an einer an der Oberfläche des Schaumes angelegten geraden Linie) von 26 Zellen pro Inch.

Alternativ kann der Schwammkörper (2) einen hydrophoben, retikulierten Polyester-Polyurethan-Schaumstoff umfassen, dessen Rohdichte gemäß ISO 845 28,0 kg/m³, dessen Stauchhärte gemäß DIN EN ISO 3386-1 3,6 kPa, dessen Bruchdehnung gemäß DIN 53571 335 %, und dessen Zellzahl (mikroskopisch bestimmt an einer an der Oberfläche des Schaumes angelegten geraden Linie) 38 Zellen pro Inch beträgt.

Gemäß einer weiteren Ausführungsform kann der Schwammkörper (2) einen hydrophoben Polyether-Polyurethan-Schaumstoff mit einer Rohdichte gemäß ISO 845 von 23,0 kg/m³, mit einer Stauchhärte gemäß DIN EN ISO 3386-1 von 3,8 kPa, mit einer Bruchdehnung gemäß DIN 53571 von 310 %und mit einer Zellzahl (mikroskopisch bestimmt an einer an der Oberfläche des Schaumes angelegten geraden Linie) von 31 Zellen pro Inch umfassen.

Es wäre auch denkbar, dass der Schwammkörper (2) zwei oder mehr konzentrisch angeordnete Lagen aus einem oder mehreren der vorgenannten Schaumstoffe umfasst.

### Figuren 3 a/b

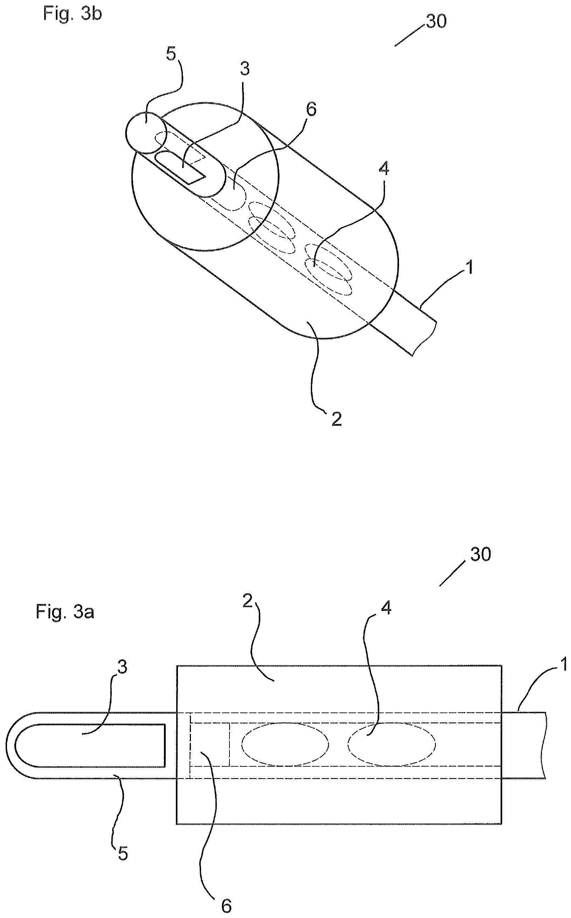

Die Figuren 3 a/b zeigen in schematisierter Darstellung eine nicht erfindungsgemäße Ausführung einer Vorrichtung zur Verwendung bei der Unterdrucktherapie von endoluminalen Wundstellen im Bereich des Gastrointestinaltraktes, bei welcher die Fassschlaufe (3) aus einem mit dem Absaugschlauch (1) unlösbar verbundenen Materialabschnitt (5) geformt ist.

Der Materialabschnitt (5) ist an seinem zum Saugkörper (30) hin zeigenden Ende zapfenartig mit dem Absaugschlauch (1) verbunden. Hierzu wird der zapfenförmige Anteil (6) des Materialabschnittes (5) herstellerseitig in den Absaugschlauch (1) eingeschoben und verklebt. Der Außendurchmesser des Materialabschnittes (5) sollte vorzugsweise dem Außendurchmesser des Absaugschlauches (1) entsprechen, so dass ein glatter Übergang vom Absaugschlauch (1) zum Materialabschnitt (5) vorhanden ist, wie in Figuren 3 a/b dargestellt. An seinem vom Saugkörper (30) weg zeigenden Ende ist an dem Materialabschnitt (5) eine Fassschlaufe (3) vorhanden. Der Materialabschnitt (5) umfasst vorzugsweise aus ein Kunststoffmaterial. Im Inneren des Materialabschnittes (5) ist vorzugsweise ein zentrales Lumen (in Figuren 3 a/b nicht dargestellt) vorhanden, welches mit dem Lumen des Absaugschlauches (1) kommunizieren kann. Hierdurch wird der im Absaugschlauch (1) vorhandene Unterdruck bis zur Fassschlaufe (3) weitergeleitet. Dies hat sich als vorteilhaft erwiesen, da hierdurch eine besonders effektive Ableitung von Wundsekreten gewährleistet werden kann. Das vom Absaugschlauch (1) weg zeigende Ende des Materialabschnittes (5) ist vorzugsweise abgerundet ausgeführt, da hierdurch eine schonende Applikation des Saugkörpers (30) in den Wundbereich erleichtert wird.

### Figur 4 a/b

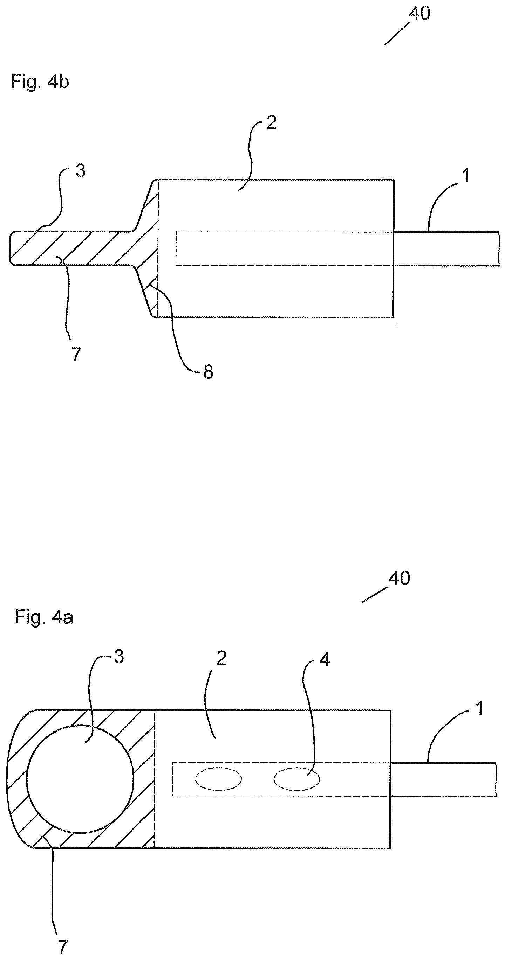

Bei dem in Figuren 4 a/b schematisch gezeigten Beispiel einer zweiten Variante der Erfindung wird die Fassschlaufe (3) herstellerseitig aus einem Abschnitt des Schwammkörpers (2) geformt. Vorzugsweise umfasst der Schwammkörper (2) einen Polymerkunststoff oder besteht aus einem Polymerkunststoff. Figur 4 a zeigt die Fassschlaufe (3) in der Aufsicht, während sie in Figur 4 b von der Seite gezeigt ist (bei der Darstellung gemäß Figur 4 b wurde der Saugkörper (40) also gegenüber Figur 4 a um 90° entlang der Schlauchachse gedreht). Die Fassschlaufe (3) wurde aus dem zylinderförmigen Schwammkörper (2) durch zwei aufeinander folgende Stanzvorgänge geformt. Bei dem ersten Stanzvorgang werden aus dem Kreiszylinder zwei Abschnitte mit kreissegmentförmigen Querschnitt gestanzt. Vorzugsweise sollte ein abgeschrägter Übergangsbereich (8) zwischen dem planaren Abschnitt (7) und dem zylinderförmiger Schwammkörper (2) vorhanden sein, wie in Figur 4 b angedeutet. Vor dem zweiten Stanzvorgang wird der Saugkörper um 90° entlang der Schlauchachse gedreht. Wird in den plattenförmig ausgebildeten Bereich (7) eine Öffnung gestanzt, um die Fassschlaufe (3) auszubilden. Die Öffnung kann beispielsweise kreisförmig, wie in Figur 4b dargestellt, ausgebildet sein. Vorzugsweise wird der die Fassschlaufe (3) umfassende Bereich (7) nach dem Stanzen gehärtet, um die Stabilität der Fassschlaufe (3) zu erhöhen. Die Härtung kann beispielsweise durch einen unter Hitzeeinwirkung durchgeführten Pressvorgang oder durch chemische Behandlung des Schaumstoffes erfolgen.

### Figur 5

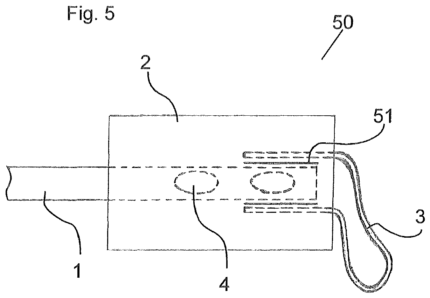

Figur 5 zeigt eine weitere nicht erfindungsgemäße Ausführung einer Vorrichtung zur Verwendung bei der Unterdrucktherapie von endoluminalen Wundstellen im Bereich des Gastrointestinaltraktes , bei welcher die Fassschlaufe (3) aus einem mit dem Absaugschlauch (1) unlösbar verbundenen Materialabschnitt, nämlich aus einem Faden (3), geformt ist. Bei dieser Ausführungsform wird die Fassschlaufe also durch einen Faden (3) gebildet, welcher durch Kleberauftrag (51) unlösbar mit der Außenseite des Absaugschlauches verbunden ist. Der Materialabschnitt (Faden 3) ist mit dem Absaugschlauch nahe seines wundnahen Endes verklebt. Der Faden ragt aus dem Schwammkörper heraus, so dass er von einem endoskopischen Instrument ergriffen werden kann.

### Figur 6

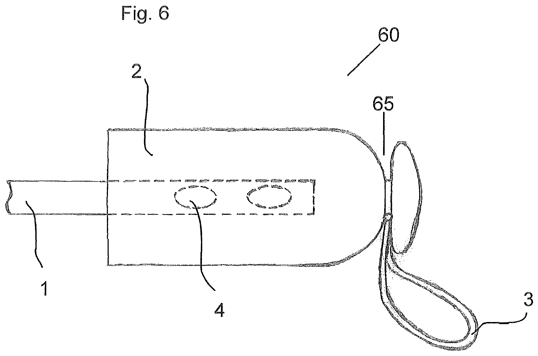

In Figur 6 ist eine weitere Möglichkeit zur Anbringung einer Fassschlaufe an einem Saugkörper (60) gezeigt (nicht erfindungsgemäße Ausführung einer Vorrichtung zur Verwendung bei der Unterdrucktherapie von endoluminalen Wundstellen im Bereich des Gastrointestinaltraktes). Hierbei weist der an dem Saugkörper (60) vorhandene Schwammkörper (2) eine Einschnürung (65) auf. An der Einschnürung (65) ist eine Fassschlaufe (3) befestigt. Bei der Fassschlaufe (3) handelt es sich um einen Faden, welcher bei Applikation des Saugkörpers (60) durch einen an einem endoskopischen Instrument vorhandenen Greifmechanismus reversibel gefasst werden kann. Die Abschnürung (65) kann durch den als Fassschlaufe (3) dienenden Faden hergestellt werden. Alternativ kann die Abschnürung (65) durch ein zusätzliches Band hergestellt werden, an dem der Faden befestigt ist.

Es wäre auch möglich, den Faden (3) durch einen zentralen Bereich des abgeschnürten (wundnahen) Endes des Schwammkörpers zu führen, so dass der Faden mittig aus dem Schwammkörper herausragt (in Figur 6 nicht dargestellt).

### Figuren 7 a/b

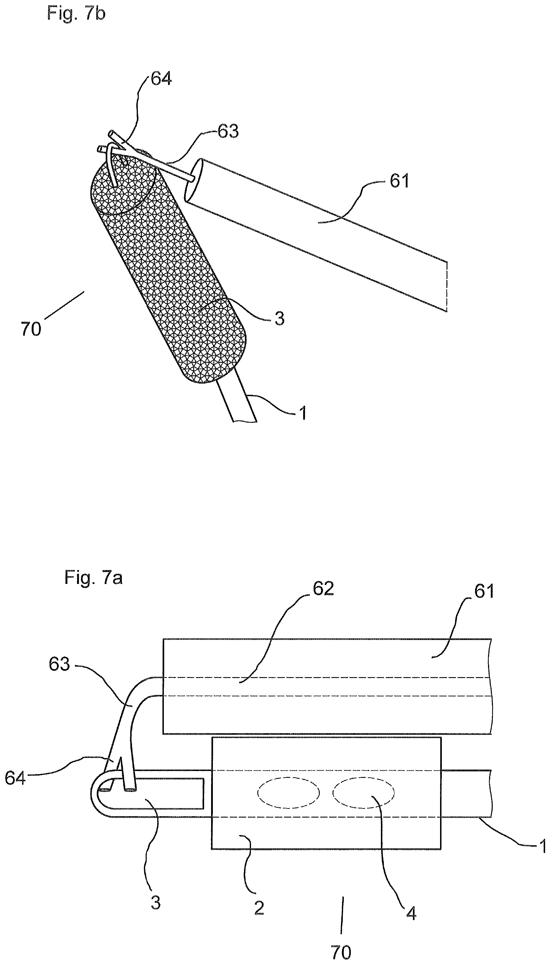

Die schematischen Darstellungen in Figur 7 zeigen einen an einem endoskopischen Instrument (61) lösbar befestigten Saugkörper (70). Wie aus Figur 7 a erkennbar, verläuft durch das endoskopische Instrument (61) ein Arbeitskanal (62), welcher zum Einführen und Bedienen von mikromechanischen Geräten vorgesehen ist. Bei dem mikromechanischen Gerät handelt es sich im Zusammenhang mit der vorliegenden Erfindung um einen Greifer mit einer abwinkelbaren Spitze. Am Ende des Greifers (63) ist eine bewegliche endoskopische Mikro-Zange (64) vorhanden. Die Mikro-Zange (64), welche in den Figuren 7 a/b nur angedeutet ist, kann mechanisch oder elektronisch ferngesteuert werden. Des Weiteren umfasst das endoskopische Instrument (61) eine Kamera (in Figur 7 nicht dargestellt) zur Spiegelung des zu behandelnden Lumen. Der endoskopische Greifer (63) mit endoskopischer Zange (64) kann den Saugkörper (70) an der Fassschlaufe (3) ergreifen. Die Verbindung von endoskopischem Instrument (61) und Saugkörper (70) mittels endoskopischer Zange (64) und Fassschlaufe (3) stellt der behandelnde Arzt noch außerhalb des Patienten her. Die Längsachse des Saugkörpers (70) kann dann parallel zur Längsachse des Endoskops (61) angeordnet werden. Beim Einführen des Endoskops (61) in das Lumen des Gastrointestinaltraktes und gegebenenfalls in einen von dem Lumen abzweigenden Abszess wird der Saugkörper "huckepack" mitgezogen. Am Zielort kann der Arzt die an dem Greifer (63) vorhandene endoskopische Zange (64) öffnen und den Saugkörper freisetzen. Anschließend wird das Endoskop (61) zurückgezogen. Nach der Entfernung des Endoskops (61) kann an den Absaugschlauch (1) Unterdruck angelegt werden. Figur 7 b zeigt eine perspektivische Darstellung von Saugkörper und Endoskop.

## Patentansprüche

1. Vorrichtung zur Verwendung bei der Unterdrucktherapie von endoluminalen Wundstellen im Bereich des Gastrointestinaltraktes, umfassend einen Schwammkörper und einen mit dem Schwammkörper unlösbar verbundenen Absaugschlauch, wobei der Schwammkörper einen Abschnitt des Absaugschlauches zumindest teilweise umschließt, und wobei zumindest an dem von dem Schwammkörper umschlossenen Abschnitt des Absaugschlauches Öffnungen vorhanden sind, **dadurch gekennzeichnet, dass** an der Vorrichtung eine bereits herstellerseitig angebrachte Fassschlaufe vorhanden ist, wobei die Fassschlaufe durch Stanzen einer Öffnung in den Schwammkörper aus einem Abschnitt des Schwammkörpers geformt ist oder durch Stanzen einer Öffnung in den Absaugschlauch aus einem Abschnitt des Absaugschlauchs geformt ist.

2. Vorrichtung nach Anspruch 1, wobei die Fassschlaufe durch Stanzen einer Öffnung in den Schwammkörper unter Hitzeeinwirkung geformt ist.

3. Vorrichtung nach Anspruch 1 oder 2, wobei die Fassschlaufe eine Länge zwischen 3 mm und 25 mm aufweist.

4. Vorrichtung nach einem oder mehreren der vorangehenden Ansprüche, wobei der Schwammkörper einen offenzelligen Schaum, insbesondere einen offenzelligen Polyurethanschaum, umfasst.

5. Vorrichtung nach einem oder mehreren der vorangehenden Ansprüche, wobei der Schwammkörper im Wesentlich zylinderförmig ausgebildet ist und eine Länge von 3 bis 10 cm sowie einen Durchmesser von 1 bis 5 cm aufweist.

6. Vorrichtung nach einem oder mehreren der vorangehenden Ansprüche, wobei der Absaugschlauch Silikon, Polyacetat, Polycarbonat, Polyethylen, Polyurethan, Weich-Polyvinylchlorid, Polyvinylalkohol, Thermoplastische Polymere oder eine Mischung daraus umfasst.

7. Vorrichtung nach einem oder mehreren der vorangehenden Ansprüche, wobei der Absaugschlauch einen Konnektor zum Verbinden des Absaugschlauches mit einer Unterdruckquelle umfasst.

8. Vorrichtung nach einem oder mehreren der vorangehenden Ansprüche, weiterhin umfassend eine Unterdruckquelle, insbesondere eine Saugflasche oder eine elektrisch antreibbare Unterdruckquelle.

9. Verfahren zur Herstellung einer Vorrichtung zur Verwendung bei der Unterdrucktherapie von endoluminalen Wundstellen im Bereich des Gastrointestinaltraktes, umfassend die Schritte
i) Bereitstellen eines für medizinische Zwecke geeigneten Schwammkörpers
ii) Bereitstellen eines für medizinische Zwecke geeigneten Absaugschlauches
iii) Herstellen einer unlösbaren Verbindung zwischen dem Schwammkörper und dem Absaugschlauch
iv) Formen einer Fassschlaufe, wobei die Fassschlaufe bereits herstellerseitig geformt wird und wobei die Fassschlaufe weiterhin entweder
- durch Stanzen einer Öffnung in den Schwammkörper aus einem Abschnitt des Schwammkörpers geformt wird, oder
- durch Stanzen einer Öffnung in den Absaugschlauch aus einem Abschnitt des Absaugschlauchs geformt wird
v) Optional Sterilisieren und Konfektionieren der Vorrichtung.

10. Kit zur Verwendung bei der Unterdrucktherapie von endoluminalen Wundstellen im Bereich des Gastrointestinaltraktes, umfassend
- eine Vorrichtung nach einem oder mehreren der Ansprüche 1 bis 7, wobei sämtliche Bestandteile der Vorrichtung steril abgepackt vorliegen
- ein Gleitgel zum Einführen des Schwammkörpers in ein Lumen des Gastrointestinaltraktes.

## Claims

1. Device for use in negative pressure therapy of endoluminal wound sites in the region of the gastrointestinal tract, comprising a sponge body and a suction hose undetachably connected to the sponge body, wherein the sponge body at least partially encloses a section of the suction hose, and wherein openings are present at least at the section of the suction hose that is enclosed by the sponge body, **characterized in that** a gripping loop already provided by the manufacturer is present on the device, wherein the gripping loop is formed from a section of the sponge body by punching an opening into the sponge body or is formed from a section of the suction hose by punching an opening into the suction hose.

2. Device according to Claim 1, wherein the gripping loop is formed by punching an opening into the sponge body under heat action.

3. Device according to Claim 1 or 2, wherein the gripping loop has a length between 3 mm and 25 mm.

4. Device according to one or more of the preceding claims, wherein the sponge body comprises an open-cell foam, especially an open-cell polyurethane foam.

5. Device according to one or more of the preceding claims, wherein the sponge body is substantially cylindrical and has a length of from 3 to 10 cm and a diameter of from 1 to 5 cm.

6. Device according to one or more of the preceding claims, wherein the suction hose comprises silicone, polyacetate, polycarbonate, polyethylene, polyurethane, soft polyvinyl chloride, polyvinyl alcohol, thermoplastic polymers or a mixture thereof.

7. Device according to one or more of the preceding claims, wherein the suction hose comprises a connector for connection of the suction hose to a negative pressure source.

8. Device according to one or more of the preceding claims, further comprising a negative pressure source, especially a suction bottle or an electrically drivable negative pressure source.

9. Method for producing a device for use in negative pressure therapy of endoluminal wound sites in the region of the gastrointestinal tract, comprising the steps of
i) providing a sponge body suitable for medical purposes
ii) providing a suction hose suitable for medical purposes
iii) establishing an undetachable connection between the sponge body and the suction hose
iv) forming a gripping loop, wherein the gripping loop is already formed by the manufacturer and wherein additionally the gripping loop either
- is formed from a section of the sponge body by punching an opening into the sponge body, or
- is formed from a section of the suction hose by punching an opening into the suction hose v) optionally sterilizing and packaging the device.

10. Kit for use in negative pressure therapy of endoluminal wound sites in the region of the gastrointestinal tract, comprising
- a device according to one or more of Claims 1 to 7, wherein all the components of the device are sterile-packed
- a lubricant gel for insertion of the sponge body into a lumen of the gastrointestinal tract.

## Revendications

1. Dispositif pour utilisation dans la thérapie par pression négative de sites de plaies endoluminales dans la région du tractus gastro-intestinal, comprenant un corps spongieux et un tuyau d'aspiration relié de manière inséparable au corps spongieux, le corps spongieux entourant au moins partiellement une section du tuyau d'aspiration, et des ouvertures étant présentes au moins sur la section du tuyau d'aspiration entourée par le corps spongieux, **caractérisé en ce qu'**une boucle de préhension déjà montée par le fabricant est présente sur le dispositif, la boucle de préhension étant formée par poinçonnage d'une ouverture dans le corps spongieux à partir d'une section du corps spongieux ou étant formée par poinçonnage d'une ouverture dans le tuyau d'aspiration à partir d'une section du tuyau d'aspiration.

2. Dispositif selon la revendication 1, dans lequel la boucle de préhension est formée par poinçonnage d'une ouverture dans le corps spongieux sous l'action de chaleur.

3. Dispositif selon la revendication 1 ou 2, dans lequel la boucle de préhension présente une longueur comprise entre 3 mm et 25 mm.

4. Dispositif selon une ou plusieurs des revendications précédentes, dans lequel le corps spongieux comprend une mousse à cellules ouvertes, en particulier une mousse de polyuréthane à cellules ouvertes.

5. Dispositif selon une ou plusieurs des revendications précédentes, dans lequel le corps spongieux est configuré sous forme essentiellement cylindrique et présente une longueur de 3 à 10 cm et un diamètre de 1 à 5 cm.

6. Dispositif selon une ou plusieurs des revendications précédentes, dans lequel le tuyau d'aspiration comprend de la silicone, du polyacétate, du polycarbonate, du polyéthylène, du polyuréthane, du polychlorure de vinyle souple, de l'alcool polyvinylique, des polymères thermoplastiques ou un mélange de ceux-ci.

7. Dispositif selon une ou plusieurs des revendications précédentes, dans lequel le tuyau d'aspiration comprend un connecteur pour relier le tuyau d'aspiration à une source de pression négative.

8. Dispositif selon une ou plusieurs des revendications précédentes, comprenant en outre une source de pression négative, en particulier une bouteille d'aspiration ou une source de pression négative pouvant être entraînée électriquement.

9. Procédé de fabrication d'un dispositif pour utilisation dans la thérapie par pression négative de sites de plaies endoluminales dans la région du tractus gastro-intestinal, comprenant les étapes suivantes :
i) la fourniture d'un corps spongieux approprié à des fins médicales,
ii) la fourniture d'un tuyau d'aspiration approprié à des fins médicales,
iii) l'établissement d'une liaison inséparable entre le corps spongieux et le tuyau d'aspiration,
iv) la formation d'une boucle de préhension, la boucle de préhension étant déjà formée par le fabricant et la boucle de préhension étant en outre soit
- formée par poinçonnage d'une ouverture dans le corps spongieux à partir d'une section du corps spongieux, soit
- formée par poinçonnage d'une ouverture dans le tuyau d'aspiration à partir d'une section du tuyau d'aspiration,
v) éventuellement la stérilisation et le conditionnement du dispositif.

10. Kit pour utilisation dans la thérapie par pression négative de sites de plaies endoluminales dans la région du tractus gastro-intestinal, comprenant :
- un dispositif selon une ou plusieurs des revendications 1 à 7, tous les constituants du dispositif se présentant sous forme emballée de manière stérile,
- un gel lubrifiant pour l'introduction du corps spongieux dans une lumière du tractus gastro-intestinal.
